# EUROPEAN PATENT APPLICATION

(11) **EP 4 850 714 A1**
(43) Date of publication of application: **09.12.2026**
(21) Application number: 25782114.0
(22) Date of filing: 28.03.2025
(51) Int. Cl.: A61M 1/16

(54) **BLOOD PURIFICATION DEVICE**

(30) Priority: 01.04.2024 JP 2024059252
(71) Applicant: Nikkiso Company Limited, Tokyo 150-6022 (JP)
(72) Inventor: USHIDA, Jun, Higashimurayama-shi, Tokyo 189-8520 (JP); HASEGAWA, Shinya, Higashimurayama-shi, Tokyo 189-8520 (JP)
(74) Representative: von Hirschhausen, Helge
(86) International application number: PCT/JP2025/012852
(87) International publication number: WO 2025/211286

(57) **Abstract**

Provide is a blood purification apparatus capable of accurately calculating the circulating-blood-volume change rate with consideration for changes in the proportion of red-blood-cell components due to a procedure performed during blood purification treatment. A blood purification apparatus is configured to purify a patient's blood by causing the blood to extracorporeally circulate through a blood circuit 1 including an arterial blood circuit 1a and a venous blood circuit 1b. The blood purification apparatus includes a hematocrit sensor 5 configured to detect a hematocrit value representing a concentration of the blood flowing in the blood circuit 1; an inputter 9 configured to accept an input of a parameter related to a procedure that causes a change in any blood parameter during blood purification treatment; and a calculator 10 configured to calculate a circulating-blood-volume change rate (ΔBV) from the hematocrit value detected by the hematocrit sensor 5 and the parameter inputted through the inputter 9.

## Description

### Technical Field

The present invention relates to a blood purification apparatus for performing blood purification treatment by purifying a patient's blood.

### Background Art

In general dialysis treatment, a blood circuit mainly constituted by flexible tubes is used to cause a patient's blood to extracorporeally circulate. The blood circuit is mainly constituted by an arterial blood circuit provided at the distal end thereof with an arterial puncture needle through which blood is collected from the patient, and a venous blood circuit provided at the distal end thereof with a venous puncture needle through which blood is returned to the patient, with a dialyzer as a blood purifier interposed between the arterial blood circuit and the venous blood circuit.

A dialysis device is provided thereinside with an ultrafiltration pump for removing water from the patient's blood so that ultrafiltration is performed in dialysis treatment. The volume of water to be removed in ultrafiltration (the ultrafiltration rate) is controlled by controlling the operation of the ultrafiltration pump. If ultrafiltration is performed abruptly or excessively, the volume of the patient's blood that is under circulation is excessively reduced, which may cause blood-pressure drop, shock, and/or the like. On the other hand, if ultrafiltration is performed slowly, the total treatment time increases, which may unnecessarily impose a burden on the patient.

Hence, in a related art, a technique of controlling the ultrafiltration rate while monitoring the patient's blood condition is proposed. Parameters and indices representing the patient's blood condition that are used in the related-art technique include hematocrit value and circulating-blood-volume change rate (ΔBV), as disclosed in PTL 1, for example. Hematocrit value is a parameter representing the concentration of blood and is specifically expressed as the volume ratio of red blood cells in total blood. Circulating-blood-volume change rate is an index that is calculated from the hematocrit value.

### Citation List

### Patent Literature

PTL 1: Japanese Unexamined Patent Application Publication No. 2007-143815

### Summary of Invention

### Technical Problem

However, the above related-art blood purification apparatus has the following problem.

For example, in a case where a blood transfusion procedure for administering a red-blood-cell product to the patient is performed during blood purification treatment, since the proportion of red-blood-cell components increases, the hematocrit value rises. In a calculation method according to the related art, no consideration is taken for changes in the proportion of red-blood-cell components due to a procedure such as blood transfusion. Therefore, the calculated circulating-blood-volume change rate (ΔBV) becomes low, resulting in an error. If such an error occurs in the circulating-blood-volume change rate (ΔBV), errors also occur in other indices (such as plasma-refill rate (PRR), for example) that are obtained using the circulating-blood-volume change rate (ΔBV) as a parameter.

The present invention has been conceived in view of the above circumstances, and an object of the present invention is to provide a blood purification apparatus capable of accurately calculating the circulating-blood-volume change rate with consideration for changes in the proportion of red-blood-cell components due to a procedure performed during blood purification treatment.

### Solution to Problem

According to an embodiment of the present invention, there is provided a blood purification apparatus configured to purify a patient's blood by causing the blood to extracorporeally circulate through a blood circuit including an arterial blood circuit and a venous blood circuit. The blood purification apparatus includes a detector configured to detect a hematocrit value representing a concentration of the blood flowing in the blood circuit; an inputter configured to accept an input of a parameter related to a procedure that causes a change in any blood parameter during blood purification treatment; and a calculator configured to calculate a circulating-blood-volume change rate from the hematocrit value detected by the detector and the parameter inputted through the inputter.

### Advantageous Effects of Invention

According to the present invention, since the circulating-blood-volume change rate is calculated from the hematocrit value detected by the detector and the parameter inputted through the inputter, the circulating-blood-volume change rate can be accurately calculated with consideration for changes in the proportion of red-blood-cell components due to a procedure performed during blood purification treatment.

### Brief Description of Drawings

[Fig. 1] Fig. 1 is a schematic diagram illustrating a blood purification apparatus according to an embodiment of the present invention.
[Fig. 2] Fig. 2 is a block diagram illustrating how major elements of the blood purification apparatus are connected.
[Fig. 3] Fig. 3 is a block diagram illustrating a calculator included in the blood purification apparatus.
[Fig. 4] Fig. 4 is a flow chart illustrating a control process that is performed in the blood purification apparatus.
[Fig. 5] Fig. 5 is a schematic diagram illustrating a method of estimating the circulating-blood-volume change rate and the plasma-refill rate in a blood purification apparatus according to another embodiment of the present invention.

### Description of Embodiments

Embodiments of the present invention will now be described specifically with reference to the drawings.

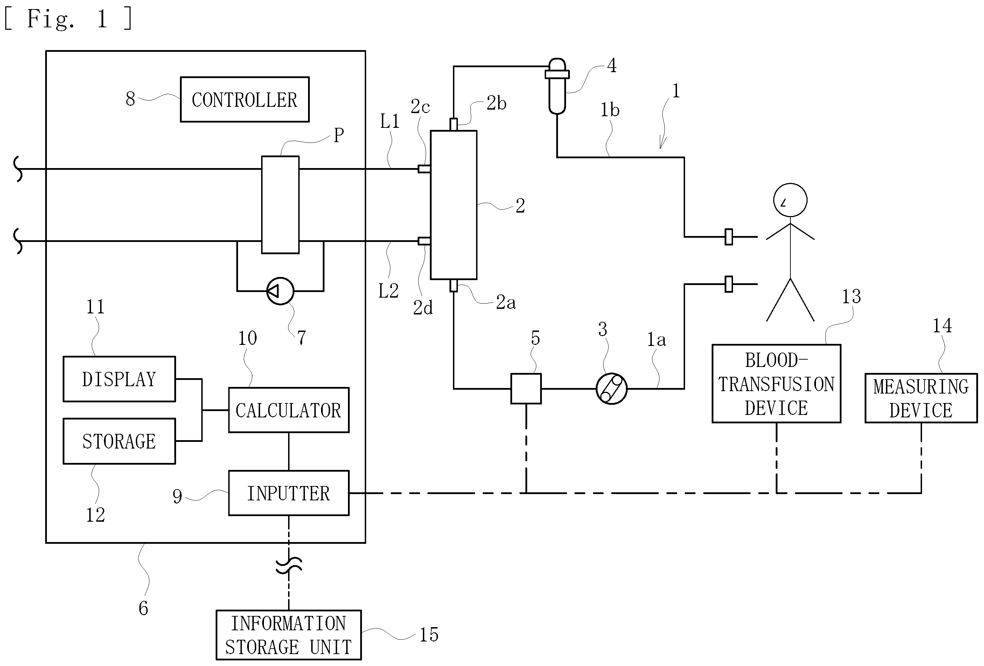

A blood purification apparatus according to the present embodiment is intended to perform blood purification treatment (for example, hemodialysis treatment) in which a patient's blood is purified while being caused to extracorporeally circulate through a blood purifier and a blood circuit. As illustrated in Fig. 1, the blood purification apparatus includes a hematocrit sensor 5 serving as a detector, a dialysis device 6, a controller 8, an inputter 9, a calculator 10, a display 11, and a storage 12.

A blood circuit 1, which is attached to the dialysis device 6, includes an arterial blood circuit 1a provided with a blood pump 3, and a venous blood circuit 1b provided with an air-trap chamber 4. A dialyzer 2 serving as a blood purification unit is connected between the arterial blood circuit 1a and the venous blood circuit 1b. The arterial blood circuit 1a and the venous blood circuit 1b are provided at the respective distal ends thereof with respective puncture needles so that the patient can be punctured.

The dialyzer 2 has a housing provided with a blood introduction port 2a, a blood delivery port 2b, a dialysate introduction port 2c, and a dialysate delivery port 2d. The arterial blood circuit 1a is connected to the blood introduction port 2a. The venous blood circuit 1b is connected to the blood delivery port 2b. The dialysate introduction port 2c and the dialysate delivery port 2d are connected to a dialysate introduction line L1 and a drain-liquid discharge line L2, respectively. The dialysate introduction line L1 and the drain-liquid discharge line L2 extend from the dialysis device 6.

The dialyzer 2 houses a plurality of hollow fibers. Spaces inside the respective hollow fibers serve as blood flow routes. Spaces between the inner peripheral surface of the housing and the outer peripheral surfaces of the hollow fibers serve as dialysate flow routes. The hollow fibers each have a number of microscopic holes (pores) extending therethrough from the outer peripheral surface to the inner peripheral surface, thereby forming hollow fiber membranes. Impurities and the like contained in the blood are allowed to permeate through the membranes into the dialysate.

The hematocrit sensor 5 (detector) is connected to the arterial blood circuit 1a. The hematocrit sensor 5 includes, for example, a light-emitting device such as an LED and a light-receiving device such as a photodiode. The light-emitting device emits light (for example, near infrared having a predetermined wavelength) to the blood, and the light-receiving device receives the light transmitted through or reflected by the blood, whereby the hematocrit value representing the concentration of the patient's blood flowing in the blood circuit 1 is measured.

The dialysis device 6 includes a duplex pump P provided astride the dialysate introduction line L1 and the drain-liquid discharge line L2, and an ultrafiltration pump 7 connected to a bypass line provided to the drain-liquid discharge line L2 in such a manner as to detour the duplex pump P. The dialysate introduction line L1 is connected at the distal end thereof to the dialyzer 2 (dialysate introduction port 2c) and at the proximal end thereof to a dialysate supply device (not illustrated) configured to prepare a dialysate at a predetermined concentration.

The drain-liquid discharge line L2 is connected at the distal end thereof to the dialyzer 2 (dialysate delivery port 2d) and at the proximal end thereof to draining means, which is not illustrated. The dialysate supplied from the dialysate supply device flows through the dialysate introduction line L1, reaches the dialyzer 2, is used for blood purification, and then flows through the drain-liquid discharge line L2 to be delivered to the draining means.

The ultrafiltration pump 7 is intended to remove water from the patient's blood flowing in the dialyzer 2. Specifically, when the ultrafiltration pump 7 is activated, the volume of fluid drained through the drain-liquid discharge line L2 becomes greater than the volume of dialysate introduced through the dialysate introduction line L1, because the duplex pump P is a metering pump. Accordingly, the excess volume of water is removed (ultrafiltered) from the blood.

When the blood pump 3 is activated, the blood collected through the arterial puncture needle flows through the arterial blood circuit 1a, reaches the dialyzer 2, is subjected to purification and ultrafiltration at the dialyzer 2, and is then returned to the patient through the venous blood circuit 1b. The supply and drainage of the dialysate, which are implemented by the duplex pump P in the present embodiment, may alternatively be implemented in another way (for example, by a chamber method).

The dialysis device 6 according to the present embodiment includes the controller 8, the inputter 9, the calculator 10, the display 11, and the storage 12. The controller 8 is, for example, a microcomputer or the like provided in the dialysis device 6 and is configured to control the operations of the blood pump 3, the duplex pump P, the ultrafiltration pump 7, and the like; the opening/closing operations of electromagnetic valves provided in the dialysis device 6; and so forth during blood purification treatment.

The inputter 9 is configured to accept inputs of parameters related to a procedure (specifically, blood transfusion, substitution, or the like performed on the patient) that causes a change in any blood parameter during blood purification treatment; and/or parameters, such as ultrafiltration rate, that are set for the treatment. In the present embodiment, the inputter 9 is connected to a blood-transfusion device 13 for performing a blood transfusion procedure on the patient during blood purification treatment, a measuring device 14 configured to measure the volume of body fluid (the volume of urination, bleeding, and/or the like) discharged outside the patient's body during blood purification treatment, and an information storage unit 15 storing the patient's personal information and so forth.

The blood-transfusion device 13 is configured to administer a blood-transfusion product to the patient. The blood-transfusion device 13 outputs, in real time, known parameters related to the concentration of blood, such as the amount of red blood cells contained in the blood-transfusion product that is administered; and progress parameters related to the blood-transfusion rate and the blood-transfusion duration. The parameters are inputted to the inputter 9 in the dialysis device 6. The measuring device 14 includes, for example, a drain provided in an ICU (intensive care unit), or the like. The measuring device 14 is configured to temporarily store the body fluid (urine and/or blood) discharged outside the patient's body and is capable of measuring the volume of the discharged body fluid. The value thus measured is outputted in real time from the measuring device 14 and is inputted to the inputter 9 in the dialysis device 6.

The information storage unit 15 is a server or the like configured to make an electronic medical chart in which personal data including the patient's weight data before blood purification treatment is recorded. In the present embodiment, a known parameter related to the patient's weight data stored in the information storage unit 15 is inputted to the inputter 9. The inputter 9 may be configured to automatically receive parameters from the blood-transfusion device 13, the measuring device 14, and the information storage unit 15, or may be configured to allow a worker to manually input parameters thereon.

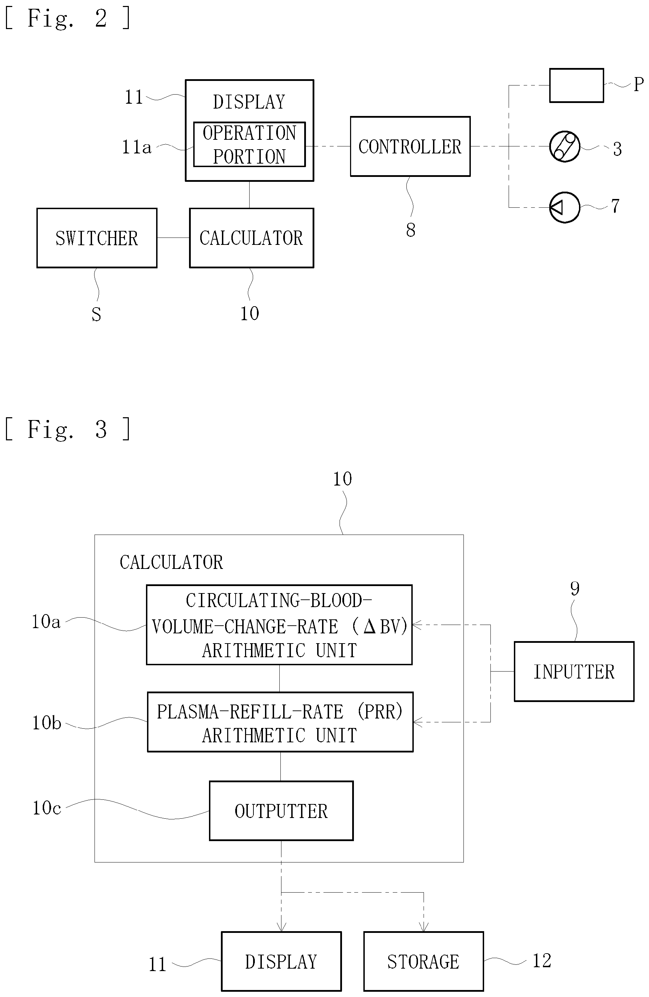

The calculator 10 is, for example, an arithmetic circuit, a microcomputer, or the like. The calculator 10 is capable of calculating the circulating-blood-volume change rate (ΔBV) and the plasma-refill rate (PRR) at every specific interval from the hematocrit value detected by the hematocrit sensor 5 (detector) and the parameters inputted through the inputter 9. Specifically, as illustrated in Fig. 3, the calculator 10 includes a circulating-blood-volume-change-rate arithmetic unit 10a connected to the inputter 9, a plasma-refill-rate arithmetic unit 10b connected to the inputter 9, and an outputter 10c.

The circulating-blood-volume-change-rate arithmetic unit 10a is an arithmetic circuit or the like configured to calculate the circulating-blood-volume change rate (ΔBV) from the parameters (known parameters and progress parameters) inputted through the inputter 9 and the hematocrit value detected by the hematocrit sensor 5. The plasma-refill-rate arithmetic unit 10b is an arithmetic circuit or the like configured to calculate the plasma-refill rate (PRR) from the parameters and the ultrafiltration rate inputted through the inputter 9 and the circulating-blood-volume change rate (ΔBV) calculated by the circulating-blood-volume-change-rate arithmetic unit 10a. The outputter 10c is configured to output the circulating-blood-volume change rate (ΔBV) calculated by the circulating-blood-volume-change-rate arithmetic unit 10a and the plasma-refill rate (PRR) calculated by the plasma-refill-rate arithmetic unit 10b to the display 11 and the storage 12.

In a case where no procedure that causes a change in any blood parameter is performed during blood purification treatment, the circulating-blood-volume change rate (ΔBV) and the plasma-refill rate (PRR) are calculated by the following mathematical expressions.

Specifically, the circulating-blood-volume change rate (ΔBV) is calculable by a mathematical expression (Ht at the start of dialysis - Ht during measurement) / Ht during measurement × 100. The plasma-refill rate (PRR) is calculable by a mathematical expression PRR = (ultrafiltration volume in measurement period + amount of change in circulating blood volume)/(measurement duration). The ratio of the volume of blood circulation to weight is 1/13 in general but may be set freely on the basis of a determination made by a medical worker such as a doctor.

When the measured hematocrit value increases, ΔBV decreases. It is said that when the value of ΔBV becomes lower than a value specific to the patient, blood-pressure drop and/or shock may occur. Hence, the hematocrit value is used as an index representing the patient's condition during ultrafiltration (not limited to ultrafiltration and encompassing all blood purification procedures). In the present embodiment, such hematocrit value is used to calculate ΔBV (circulating-blood-volume change rate), and the result is used as an index representing the patient's condition.

In a case where a procedure that causes a change in any blood parameter is performed during blood purification treatment (for example, a blood transfusion procedure is performed by the blood-transfusion device 13 during blood purification treatment), known parameters related to the concentration of blood that is transfused by the blood-transfusion device 13, such as the hematocrit value or hemoglobin concentration of the blood-transfusion product that is introduced into the patient's body, and progress parameters related to the rate and duration of blood transfusion that is performed by the blood-transfusion device 13 are inputted to the inputter 9, and the circulating-blood-volume change rate (ΔBV) is calculated through the following calculation performed by the calculator 10. The following is based on a premise that ΔBV is successively calculated at every specific interval.

First, using the hematocrit value of the blood-transfusion product (Htin (%)), the blood-transfusion rate (ml/min), and the blood-transfusion duration (min) that have been inputted to the inputter 9, the volume of red blood cells administered to the patient (VRBCin (g)) is calculated through Mathematical Expression (1) below. 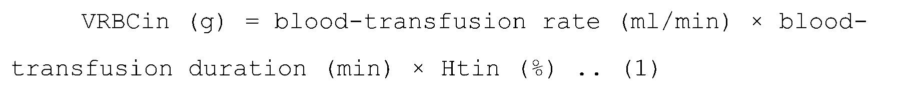 VRBCin (g) = blood-transfusion rate (ml/min) × blood-transfusion duration (min) × Htin (%) (1)

Subsequently, an increment in the hematocrit value due to blood transfusion (HtA (%)) is calculated through Mathematical Expression (2) below. 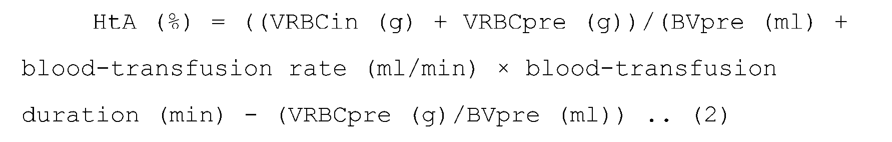 HtA (%) = ((VRBCin (g) + VRBCpre (g))/(BVpre (ml) + blood-transfusion rate (ml/min) × blood-transfusion duration (min) - (VRBCpre (g)/BVpre (ml)) (2)

Furthermore, ΔBVa (%) ignoring the estimated increment in the volume of red blood cells due to blood transfusion is calculated through Mathematical Expression (3) below. $ΔBVa \left(%\right) = \left(Ht0 \left(%\right)/\left(Htt \left(%\right)-HtA \left(%\right)\right)-1\right) \times 100$ (3)

Subsequently, ΔBV (%) in consideration of blood transfusion is calculated through Mathematical Expression (4) below. 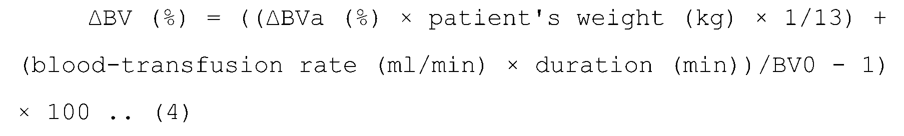 ΔBV (%) = ((ΔBVa (%) × patient's weight (kg) × 1/13) + (blood-transfusion rate (ml/min) × duration (min))/BV0 - 1) × 100 (4)

In the above expressions, Htin denotes the hematocrit value (%) of the blood product administered, VRBCin denotes the volume (g) of red blood cells administered, VRBCpre (g) denotes Htpre (%) (last calculated Ht (%)) × BVpre (g) (last calculated BV (g)), BVpre (%) denotes the patient's weight × 1/13 × last calculated ΔBV (%), ΔBVa (%) denotes ΔBV (%) ignoring the error due to the increase in red blood cells, BV0 denotes the patient's weight (kg) × 1/13 × ΔBV (%) at the start of treatment, Ht0 denotes the hematocrit value (%) at the start of treatment, Htt denotes the hematocrit value (%) during measurement, and HtA denotes the estimated increment in the hematocrit value (%) due to blood transfusion.

The hematocrit value (%) of the blood product administered may be converted through Mathematical Expression (5) below. $Htin \left(%\right) = Hbin \left(g/dl\right) \times 3.3$ (5) where Hbin denotes the hemoglobin concentration (g/dl) of the blood product administered.

Subsequently, the calculator 10 calculates the plasma-refill rate (PRR) by using the circulating-blood-volume change rate (ΔBV) calculated through Mathematical Expression (4) above and the parameters inputted from the measuring device 14 (the parameters related to the volume of body fluid discharged outside the patient's body during blood purification treatment, and the ultrafiltration rate).

That is, the inputter 9 according to the present embodiment is configured to accept inputs of not only parameters related to a procedure that causes a change in any blood parameter during blood purification treatment but also parameters related to the volume of the body fluid discharged outside the patient's body and/or the volume of any fluid (such as an infusion fluid and/or a substitution fluid) introduced into the patient's body during blood purification treatment and the ultrafiltration rate, and the calculator 10 is configured to calculate the plasma-refill rate (PRR) from the circulating-blood-volume change rate (ΔBV) and the parameters and ultrafiltration rate inputted through the inputter 9.

Plasma refill refers to a function exerted in response to a reduction in the circulating blood volume so that the circulating blood volume is maintained with the movement of water from the outside of blood vessels to the inside of blood vessels. The power that causes plasma refill is believed to be the difference in colloid osmotic pressure and the difference in hydrostatic pressure between the inside and the outside of blood vessels. The speed of plasma refill is referred to as plasma-refill rate (also referred to as plasma-replacement rate). If the rate of ultrafiltration by the ultrafiltration pump 7 is equal to the plasma-refill rate, substantially no change occurs in the circulating-blood-volume change rate (ΔBV), theoretically. Therefore, in general, a dialysis treatment that imposes less burden on the patient is achieved.

The calculator 10 according to the present embodiment calculates the plasma-refill rate (PRR) through Mathematical Expression (6) below. 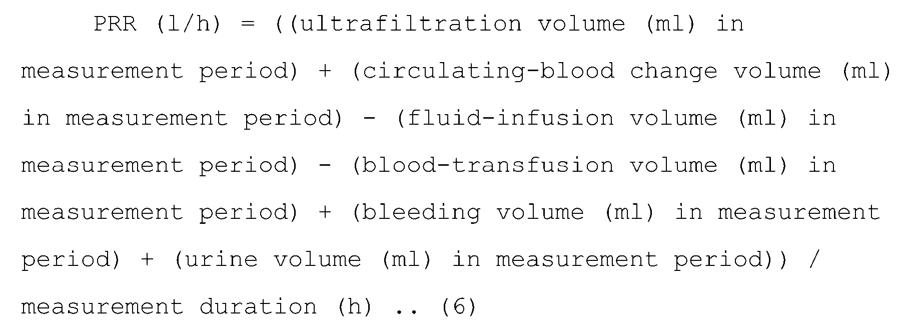 PRR (l/h) = ((ultrafiltration volume (ml) in measurement period) + (circulating-blood change volume (ml) in measurement period) - (fluid-infusion volume (ml) in measurement period) - (blood-transfusion volume (ml) in measurement period) + (bleeding volume (ml) in measurement period) + (urine volume (ml) in measurement period)) / measurement duration (h) (6)

The measurement period refers to a period of time over which ΔBV is calculated successively.

The display 11 is a liquid-crystal monitor or the like configured to display set values of the devices and the patient's condition during blood purification treatment. In the present embodiment, the display 11 displays in real time the circulating-blood-volume change rate (ΔBV) and the plasma-refill rate (PRR) calculated by the calculator 10. The storage 12 is a memory or the like configured to store the circulating-blood-volume change rate (ΔBV) and the plasma-refill rate (PRR) calculated by the calculator 10.

As illustrated in Fig. 2, the display 11 according to the present embodiment includes an operation portion 11a. A switcher S is connected to the calculator 10. The operation portion 11a is a site (including input buttons or the like displayed on the screen) displayed in a portion of the screen of the display 11, which is a touch panel, for example. When a medical worker visually recognizes the circulating-blood-volume change rate (ΔBV) or plasma-refill rate (PRR) displayed on the screen, the medical worker operates the operation portion 11a. Thus, the medical worker can operate the duplex pump P, the blood pump 3, and/or the ultrafiltration pump 7, or the like via the controller 8. The operation portion 11a may include mechanical switches instead of input buttons or the like displayed on the screen of the display 11.

The switcher S is configured to allow the operator to perform an operation thereon to make an input for switching between the display by the display 11 and the calculation by the calculator 10 with a given timing. The switcher S need only allow the operator to perform an input operation thereon with a given timing and may be, for example, a switch part displayed on a touch panel (including the display 11) or a mechanical switch, as with the operation portion 11a.

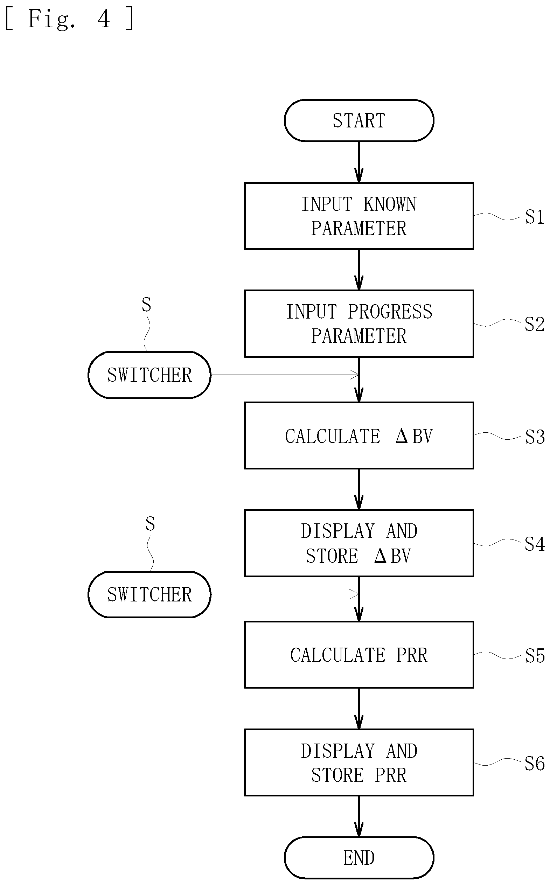

Now, a control process to be performed by the controller 8 of the blood purification apparatus according to the present embodiment in a case where blood transfusion is performed as a procedure that causes a change in any blood parameter will be described with reference to a flow chart illustrated in Fig. 4.

First, through the inputter 9, the known parameters (the hematocrit value of the blood-transfusion product, the patient's weight before the treatment, and so forth) related to the concentration of blood that is transfused by the blood-transfusion device 13 are inputted in step S1, and the progress parameters related to the rate and duration of blood transfusion that is performed by the blood-transfusion device 13 are inputted in step S2. Then, on condition that an input operation on the switcher S is performed by the operator, the process proceeds to step S3, where the calculator 10 calculates ΔBVa by subtracting the increment in the hematocrit value (HtA) due to blood transfusion from the hematocrit value (Ht) detected by the hematocrit sensor 5, and calculates the circulating-blood-volume change rate (ΔBV) by adding the increment in the blood volume due to blood transfusion.

Subsequently, the process proceeds to step S4, where the circulating-blood-volume change rate (ΔBV) calculated by the calculator 10 is displayed on the display 11 and is stored in the storage 12. Furthermore, on condition that an input operation on the switcher S is performed by the operator, the process proceeds to step S5, where the plasma-refill rate (PRR) is calculated from the patient's weight before the treatment, the circulating-blood-volume change rate (ΔBV) calculated in step S3, and parameters (the volume of urine, blood, and/or the like discharged from the patient) inputted on a time-course basis from the measuring device 14 to the inputter 9. Then, in step S6, the calculated plasma-refill rate (PRR) is displayed on the display 11 and is stored in the storage 12.

While the present embodiment has been described above, the present invention is not limited thereto. For example, the procedure that causes a change in any blood parameter during blood purification treatment is not limited to fluid infusion and/or substitution and may be any other procedure. Furthermore, the parameters to be used in calculating the circulating-blood-volume change rate (ΔBV) and the plasma-refill rate (PRR) may be any other parameters.

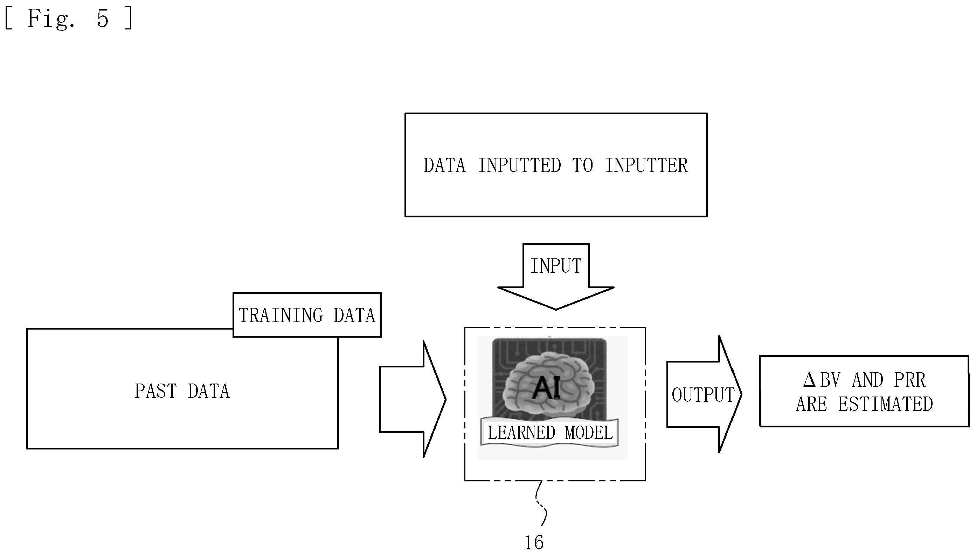

Furthermore, as a blood purification apparatus according to another embodiment of the present invention, the apparatus may include an estimator 16. As illustrated in Fig. 5, using a learned model (generative AI) created through machine learning, the estimator 16 estimates the circulating-blood-volume change rate (ΔBV) and the plasma-refill rate (PRR) from the hematocrit value detected by the hematocrit sensor 5 (detector) and the parameters inputted to the inputter 9. Specifically, the estimator 16 acquires accumulated data by accumulating a plurality of sets of hematocrit values detected by the hematocrit sensor 5 (detector) and parameters inputted to the inputter 9 in past treatments, and then-calculated circulating-blood-volume change rates (ΔBV) and plasma-refill rates (PRR). Furthermore, the estimator 16 uses the accumulated data as training data and thus obtains a learned model by performing machine learning in which the circulating-blood-volume change rate (ΔBV) and the plasma-refill rate (PRR) are estimated.

When input data for the inputter 9 (that is, the known parameters and the progress parameters inputted to the inputter 9) is inputted in the current treatment, the estimator 16 can estimate the circulating-blood-volume change rate (ΔBV) and the plasma-refill rate (PRR) by using the learned model obtained through the above machine learning. Machine learning refers to a technology related to an artificial intelligence (generative AI) in which a computer learns a huge amount of data and automatically generates algorithms and models for performing tasks such as classification and prediction. Technologies and algorithms that enable machine learning include, for example, a neural network.

According to a first aspect of the present invention, there is provided a blood purification apparatus configured to purify a patient's blood by causing the blood to extracorporeally circulate through a blood circuit 1 including an arterial blood circuit 1a and a venous blood circuit 1b. The blood purification apparatus includes a hematocrit sensor 5 (detector) configured to detect a hematocrit value representing a concentration of the blood flowing in the blood circuit 1; an inputter 9 configured to accept an input of a parameter related to a procedure that causes a change in any blood parameter during blood purification treatment; and a calculator 10 configured to calculate a circulating-blood-volume change rate (ΔBV) from the hematocrit value detected by the hematocrit sensor 5 and the parameter inputted through the inputter 9. Since the circulating-blood-volume change rate (ΔBV) is calculated from the hematocrit value detected by the detector and the parameter inputted through the inputter, the circulating-blood-volume change rate (ΔBV) can be accurately calculated with consideration for changes in the proportion of red-blood-cell components due to a procedure performed during blood purification treatment.

According to a second aspect of the present invention, in the first aspect, the procedure that causes a change in any blood parameter is blood transfusion or substitution performed on the patient. Therefore, errors due to blood transfusion or substitution can be absorbed, and the circulating-blood-volume change rate (ΔBV) can be accurately calculated.

According to a third aspect of the present invention, in the first aspect, the inputter 9 is connected to a blood-transfusion device 13 configured to transfuse blood to the patient during blood purification treatment and accepts inputs of a known parameter related to a concentration of the blood transfused by the blood-transfusion device 13 and a progress parameter related to a rate and duration of blood transfusion performed by the blood-transfusion device 13, and the calculator 10 calculates the circulating-blood-volume change rate (ΔBV). Therefore, even if fluid infusion is performed during blood purification treatment, the circulating-blood-volume change rate (ΔBV) can be accurately calculated.

According to a fourth aspect of the present invention, in the first aspect, the inputter 9 accepts inputs of not only the parameter related to the procedure that causes a change in any blood parameter during blood purification treatment but also a parameter related to a volume of body fluid discharged outside the patient's body and/or a volume of any fluid introduced into the patient's body during blood purification treatment; and an ultrafiltration rate, and the calculator 10 calculates a plasma-refill rate (PRR) from the circulating-blood-volume change rate (ΔBV) and the parameters and the ultrafiltration rate inputted through the inputter 9. Therefore, the plasma-refill rate (PRR) can be accurately calculated with consideration for changes in the proportion of red-blood-cell components due to any procedure performed during blood purification treatment.

According to a fifth aspect of the present invention, in the fourth aspect, the inputter 9 is connected to an information storage unit 15 to which personal data including weight data of the patient before blood purification treatment is inputted and to a measuring device 14 configured to measure the volume of body fluid discharged outside the patient's body during blood purification treatment, and accepts inputs of a known parameter related to the weight data of the patient stored in the information storage unit 15; a progress parameter related to the volume of body fluid measured by the measuring device 14; and the ultrafiltration rate, and the calculator 10 calculates the plasma-refill rate (PRR). Therefore, the plasma-refill rate (PRR) can be accurately calculated.

According to a sixth aspect of the present invention, the blood purification apparatus according to the first aspect further includes an estimator 16 configured to estimate the circulating-blood-volume change rate from the hematocrit value detected by the hematocrit sensor 5 (detector) and the parameters inputted through the inputter 9 by using a learned model obtained through machine learning. Therefore, the circulating-blood-volume change rate can be accurately determined by using a learned model created by an artificial intelligence (AI).

### Industrial Applicability

The present invention is also applicable to any blood purification apparatus having a different appearance, additional functions, and/or the like within the spirit of the present invention.

### Reference Signs List

- 1: blood circuit
- 1a: arterial blood circuit
- 1b: venous blood circuit
- 2: dialyzer
- 2a: blood introduction port
- 2b: blood delivery port
- 2c: dialysate introduction port
- 2d: dialysate delivery port
- 3: blood pump
- 4: air-trap chamber
- 5: hematocrit sensor (detector)
- 6: dialysis device
- 7: ultrafiltration pump
- 8: controller
- 9: inputter
- 10: calculator
- 11: display
- 12: storage
- 13: blood-transfusion device
- 14: measuring device
- 15: information storage unit
- 16: estimator
- P: duplex pump
- L1: dialysate introduction line
- L2: drain-liquid discharge line

## Claims

1. A blood purification apparatus configured to purify a patient's blood by causing the blood to extracorporeally circulate through a blood circuit including an arterial blood circuit and a venous blood circuit, the blood purification apparatus comprising:
a detector configured to detect a hematocrit value representing a concentration of the blood flowing in the blood circuit;
an inputter configured to accept an input of a parameter related to a procedure that causes a change in any blood parameter during blood purification treatment; and
a calculator configured to calculate a circulating-blood-volume change rate from the hematocrit value detected by the detector and the parameter inputted through the inputter.

2. The blood purification apparatus according to claim 1, wherein the procedure that causes a change in any blood parameter is blood transfusion or substitution performed on the patient.

3. The blood purification apparatus according to claim 1, wherein the inputter is connected to a blood-transfusion device configured to transfuse blood to the patient during blood purification treatment and accepts inputs of a known parameter related to a concentration of the blood transfused by the blood-transfusion device and a progress parameter related to a rate and duration of blood transfusion performed by the blood-transfusion device, and the calculator calculates the circulating-blood-volume change rate.

4. The blood purification apparatus according to claim 1, wherein the inputter accepts inputs of not only the parameter related to the procedure that causes a change in any blood parameter during blood purification treatment but also a parameter related to a volume of body fluid discharged outside the patient's body and/or a volume of any fluid introduced into the patient's body during blood purification treatment; and an ultrafiltration rate, and the calculator calculates a plasma-refill rate from the circulating-blood-volume change rate and the parameters and the ultrafiltration rate inputted through the inputter.

5. The blood purification apparatus according to claim 4, wherein the inputter is connected to an information storage unit to which personal data including weight data of the patient before blood purification treatment is inputted and to a measuring device configured to measure the volume of body fluid discharged outside the patient's body during blood purification treatment, and accepts inputs of a known parameter related to the weight data of the patient stored in the information storage unit; a progress parameter related to the volume of body fluid measured by the measuring device; and the ultrafiltration rate, and the calculator calculates the plasma-refill rate.

6. The blood purification apparatus according to claim 1, further comprising an estimator configured to estimate the circulating-blood-volume change rate from the hematocrit value detected by the detector and the parameters inputted through the inputter by using a learned model obtained through machine learning.
